# EUROPEAN PATENT APPLICATION

(11) **EP 4 847 628 A1**
(43) Date of publication of application: **02.12.2026**
(21) Application number: 25179773.4
(22) Date of filing: 30.05.2025
(51) Int. Cl.: C12Q 1/6844

(54) **ELECTRICAL CONTROL OF ENZYMATIC ACTIVITY FOR BIOPOLYMER SYNTHESIS**

(71) Applicant: Imec VZW, 3001 Leuven (BE)
(72) Inventor: Willems, Kherim, 3051 Sint-Joris-Weert (BE); Van Dorpe, Pol, 3510 Spalbeek (BE)
(74) Representative: Roth, Sebastian

(57) **Abstract**

The present disclosure relates to a device and a method for controlling enzymatic activity. The device comprises a solid support substrate, and an electrically controlled element arranged on the support substrate. At least a first enzyme and a second enzyme are electrically coupled to the electrically controlled element. The enzymes respectively comprise at least one redox-active component. A first voltage profile applied to the first enzyme causes a change in the enzymatic activity of the first enzyme, due to a change in the redox state of the redox-active component of the first enzyme. A second voltage profile applied to the second enzyme, which is different to the first voltage profile, causes a change in the enzymatic activity of the second enzyme, due to a change in the redox state of the redox-active component of the second enzyme.

## Description

### TECHNICAL FIELD

The present disclosure relates to a device and a method for controlling enzymatic activity. For example, the device and method may be used to control biopolymer synthesis, especially DNA synthesis. To this end, the device comprises electrically controllable enzymes to perform the synthesis.

### BACKGROUND

Hybrid nanotechnology-created by combining molecular machines, like enzymes, and nanoscale solid-state devices, like electrodes-holds the key to enabling a wide array of applications. These applications range from miniaturized biosensors (e.g., nanopore DNA sequencing, or transistor-based biomarker detection) to molecular computing (e.g., molecular circuits) and molecular machines for synthesis (e.g., high-throughput de novo DNA synthesis).

While fluorescence and ionic current sensing techniques are the two most used methods to transduce (bio)chemical events into electrical signals, using these techniques for exerting nanoscale control for more complex, programmable synthesis results in significant scaling and addressability challenges. In contrast, a method that enables direct electrical control and interrogation of single molecules, particularly of the enzyme(s) involved in the synthesis, could provide a more flexible, scalable, and programmable approach. However, methodologies for achieving precise, real-time electrochemical control over enzymatic activity of an enzyme are currently underdeveloped.

Conventional high-throughput *de novo* DNA synthesis approaches typically rely on the sequential flushing or depositing of distinct reagents (e.g., nucleotide precursors, deblocking agents, and washing solutions) onto a micro-structured surface. This "temporal" compartmentalization allows multiplexed writing of arbitrary DNA sequences at specific locations, often enabled by the selective presence of nucleotide precursors combined with site-selective removal of protective groups (e.g., via photolithography or locally electrochemically generated acid) or site-selective reaction activation. However, these sequential methods suffer from limitations in speed, reagent consumption, and difficult real-time control.

In particular, sequential flushing typically requires multiple steps (e.g., nucleotide addition, deblocking, washing) for each base incorporation across an array, which leads to substantial reagent waste and high costs. Further, the conventional methods suffer from limited real-time control over the enzymatic activity at individual enzyme sites (e.g., synthesis sites).

### SUMMARY

In view of the above, this disclosure has the objective to provide a device and method for controlling enzymatic activity. An objective is to specifically enable a precise spatiotemporal control (e.g., activation/deactivation of a polymerase) and real-time monitoring of the enzymatic activity (e.g., polymerase activity). Another objective is to enable a "one-pot" approach, wherein all necessary reagents (e.g., for performing DNA synthesis, all four nucleotide types) are present simultaneously in a fluidic reservoir. An objective in this regard is to avoid the need for sequential flushing when performing synthesis using enzymes, and consequently dramatically reducing the reagent consumption and associated costs. Another objective is to enable a future integration of additional electrochemically controlled enzymatic activity.

These and other objectives are achieved by the solutions of this disclosure, which are described in the independent claims. Advantageous implementations are described in the dependent claims.

A first aspect of this disclosure provides a device for controlling enzymatic activity, the device comprising: a solid support substrate; an electrically controlled element arranged on the support substrate; wherein at least a first enzyme and a second enzyme are electrically coupled to the electrically controlled element; wherein each enzyme comprises at least one redox-active component; wherein a first voltage profile applied to the first enzyme causes a change in the enzymatic activity of the first enzyme; and wherein a second voltage profile applied to the second enzyme, which is different to the first voltage profile, causes a change in the enzymatic activity of the second enzyme.

The change in the enzymatic activity of the first enzyme is due to a change in the redox state of the redox-active component of the first enzyme, when the first voltage profile is applied. The change in the enzymatic activity of the second enzyme is due to a change in the redox state of the redox-active component of the second enzyme, when the second voltage profile is applied. The solid substrate may be a silicon- or glass-based support.

The device of the first aspect utilizes the redox-active enzymes, for example, engineered polymerases, which are individually addressable via the electrically controlled element. In this way, a precise control of the enzymatic activity of the enzymes is achieved. The control is shifted from fluidics (as conventionally done by sequential flushing) to site-specific electrochemical activation and/or deactivation of the enzymes. As a consequence, reagents must no longer be continuously exchanged and hence their consumption and associated costs are dramatically reduced when, for example, performing synthesis using the enzymes. The device of the first aspect is particularly suitable for controlling DNA synthesis, although the described principles may be used for other enzyme-based operations and nanoscale machines as well (e.g., transcription-translation of mRNA and proteins, nucleic-acid assembly, energy harvesting, reagent regeneration, etc.).

An enzyme is a molecular machine, natural or artificial, that is capable of performing a specific action at a molecular level.

A redox-active component is a molecular component that contains a redox-sensitive element whose oxidation state results in a measurable change in the activity or conformation of the molecular component or the enzyme it is attached to or interacts with.

In an implementation of the device, the electrically controlled element comprises an electrode; the first enzyme and the second enzyme are attached to the electrode; and the first voltage profile and the second voltage profile are respectively selected based on intrinsic redox potentials of the respective redox-active components of the first enzyme and the second enzyme.

The enzymes may specifically have different intrinsic redox potentials. Each enzyme can hence be switched individually. There may be multiple enzymes, including the first and second enzyme.

In an implementation of the device, the electrically controlled element comprises an electrode pair of a first electrode and a second electrode, which are separated by a gap; a molecular structure bridges the gap and is attached to the first and the second electrode; and the molecular structure comprises at least one molecular wire and the first enzyme and/or the second enzyme attached to the at least one molecular wire.

In an implementation of the device, the first enzyme and the second enzyme are attached to the same molecular wire; and the first and the second voltage profile are respectively selected based on a respective position of the first enzyme and the second enzyme on said molecular wire.

For example, the enzymes can have the same intrinsic redox potentials, but their effective switching potential is respectively determined by the position of the enzyme on the molecular wire (voltage-divider effect). There may be multiple enzymes, including the first and second enzyme.

Each part of the molecular wire may act as a voltage divider, meaning that each enzyme on the molecular wire will experience a different potential. Hence, even if the redox sites in each enzyme have the same intrinsic redox potential, the potential required between the electrodes will be different.

The electrically controlled element, which may comprise the electrode or electrode pair mentioned above, may be a part of one pixel of multiple pixels of the device, for instance, multiple synthesis pixels. Each pixel of the device may comprise a similar electrically controlled element, which may respectively include at least one electrode or at least one electrode pair.

The gap between the first and the second electrode of any electrode pair may be in a range of 10 nm to 100 nm, or 5 nm to 50 nm, or 2 nm to 20 nm. Thus, the electrode pair may be referred to as a nano-gap electrode pair. The conductive molecular wire may allow bypassing the potential drop due to an electrical double layer, resulting in a lower voltage requirement, and increasing the specificity.

In an implementation of the device, the first and/or second voltage profile applied to the first and second electrode respectively oxidizes or reduces the redox-active component of the first enzyme and/or the second enzyme, which causes a change in the enzymatic activity of the respective enzyme.

In an implementation of the device, the device further comprises a controller configured to apply the first voltage profile and/or the second voltage profile to the first enzyme and/or the second enzyme via the electrically controlled element.

In an implementation of the device, the controller is configured to: apply, as a part of the first voltage profile, a first voltage difference between the first and second electrode to pass a current over the molecular structure, which oxidizes the redox-active component of the first enzyme; apply, as a part of the second voltage profile, a second voltage difference between the first and second electrode to pass a current over the molecular structure, which reduces the redox-active component of the first enzyme; and apply a third voltage difference between the first and second electrode to measure a current flowing over the molecular structure, or optically monitor the enzymatic activity of the first enzyme.

The above implementation is described in relation to the first enzyme, but the same implementation is possible with the second enzyme. The controller can electrically control the enzymatic activity of the first enzyme and/or second enzyme, for instance, for performing molecular synthesis. The current can pass through the molecular wire, so as to ensure that only the needed redox site of the respective enzyme is activated. For example, the first voltage difference can be selectively used to activate the first enzyme, the second voltage difference can be used to deactivate the first enzyme, and the third voltage difference can be used to measure the current to monitor the first enzyme. The third voltage difference can be the same as the voltage difference that was used to generate the required state. The second enzyme may be likewise controlled and monitored with suitable voltage differences. The first enzyme and the second enzyme can be individually controlled by the respective voltage profiles applied to them via the electrically controlled element. This is possible also, if the enzymes are connected to the same electrode, electrode pair, or molecular wire, of the electrically controlled element.

In an implementation of the device, the controller is further configured to determine a current pattern in the measured current flowing over the molecular structure, wherein the current pattern is indicative of the enzymatic activity of the first enzyme.

This ability of the controller to detect current patterns, allows monitoring the catalytic activity of the enzyme (e.g., by detecting conformation changes within the enzyme).

In an implementation of the device, the molecular structure comprises one molecular wire bridging the gap and attached to both the first and second electrode; and the first and/or the second enzyme is attached to the molecular wire between the first and second electrode.

In one exemplary configuration, a single enzyme may be attached to the molecular wire. In another exemplary configuration two or even more enzymes may be attached to the molecular wire.

In an implementation of the device, the molecular structure comprises two or more molecular wires, each molecular wire being connected with one of its ends to one of the first and second electrode; and the first and/or the second enzyme is attached between the first and second electrode to the respective other ends of at least one molecular wire.

In one exemplary configuration, a single enzyme may be arranged between each two molecular wires, i.e., one or more chains of molecular wire-enzyme-molecular wire may be formed. In another exemplary configuration two or more enzymes may be attached between each two molecular wires.

The above implementations describe alternative configurations of molecular structures bridging the gap between the two electrodes.

In an implementation of the device, the first and second electrode comprise a material configured to facilitate electron transfer across the interface between the material and the respective molecular wire.

For instance, the material of the electrodes may be a metal material. The material of the electrodes may also be a conductive material including carbon nanotubes (CNTs), organic semiconductors, 2D materials, etc. The metal material may, for example, be Pt, Au, Ru, Pd, Ir, TiN, or IrOₓ, or may comprise any of these metal materials.

In an implementation of the device, at least one molecular wire comprises a DNA, or a peptide-based molecule, or a protein.

Generally, any suitable molecular wire may be used, but a DNA as the molecular wire may have some benefits, for example, it has also useful properties for facilitating site-specific immobilization. The molecular wire may also be modified-e.g., silicate or metalized-to increase its stability and/or conductivity.

In an implementation of the device, the first enzyme and/or the second enzyme is a polymerase, for example, a redox-active polymerase comprising a redox-active aptamer.

In an implementation of the device, the controller is configured to: apply the first voltage difference with an oxidative potential between the first and second electrode to activate the polymerase; and/or apply the second voltage difference with a reductive potential between the first and second electrode to deactivate the polymerase.

In this way, the controller can control the enzymatic activity of the polymerase precisely and in real time.

In an implementation of the device, the polymerase has an affinity for a specific nucleotide.

The polymerase affinity to the specific nucleotide may indicate how strongly the polymerase binds and incorporates that nucleotide, in particular, stronger than to other nucleotides.

In an implementation of the device, the molecular structure further comprises a synthetic single-stranded pseudo-template strand attached to the polymerase within an active site of the polymerase; and the single-stranded pseudo-template strand comprises a single natural base, which provides the polymerase with the affinity for the specific nucleotide.

In an example, the pseudo-template strand may contain the single natural base at the 5' end, optionally followed by several (0 to 5) universal bases at the 3' end. The natural base provides the base-specificity of the polymerase. The universal bases provide a stabilization of an incoming primer strand by temporary hybridization. The universal nature of those bases allows them to pair with any incoming strand.

In an implementation of the device, the current pattern is indicative of a successful incorporation by the polymerase of a nucleotide into a primer strand.

For example, the primer strand may be immobilized on the solid substrate of the device in the proximity of the electrode element.

In an implementation of the device, the electrically controlled element is connected to a plurality of enzymes comprising the first enzyme and the second enzyme; and each enzyme comprises a different polymerase with a different affinity for a specific nucleotide than the other polymerases.

For example, in an implementation, the electrically controlled element may comprise at least four electrode pairs. In this exemplary implementation, each of the four electrode pairs is connected to one molecular structure, wherein the molecular structure comprises at least one conductive molecular wire and at least one enzyme with a redox-active component attached to at least one molecular wire. The respective molecular structure bridges the gap between the two electrodes of that electrode pair. The enzymes of the molecular structures each comprise a polymerase with a different affinity for a specific nucleotide.

In a particular implementation, different redox-activated polymerases may be used, which have different potential windows (e.g., [-0.1 V, +0.1 V], [-0.2 V, +0.2 V], [-0.3 V, +0.3 V], [-0.4 V, +0.4 V]). A specific activation may be achieved by a pulse of an activation voltage, followed by a pulse of a deactivation voltage for all polymerases which were activated by the first pulse. For example, specifically activation to the last-mentioned polymerase [-0.4 V, +0.4V] may be achieved by an activation pulse at +0.4V (which activates ALL enzymes), followed by a deactivation pulse at -0.3V (which deactivates the enzymes at 0.1, 0.2, and 0.3V). Note that this deactivation pulse preferably follows quickly after the activation pulse, and thus beneficially deactivates the enzymes faster than their reaction rate to prevent errors. A modulation of a potential window for the charge transfer can be achieved by changing the charge transfer resistance ("stretching out" the voltage window), for example by increasing the length of the molecular wire.

If the first and the second enzyme are attached to the same molecular wire at different positions (e.g., along the molecular wire between the two electrodes), a voltage divider effect may be employed, which leads to each of the two enzymes experiencing a different potential for a particular applied voltage. This can be used to selectively and independently address each enzyme, for instance, to activate or deactivate the enzyme. For example, to activate only the first enzyme, which may have the same potential window of [-0.2 V, +0.2 V] than the second enzyme, a +0.3V pulse may be applied. This pulse may result in +0.2V experienced by the first enzyme (due to series resistance), which is sufficient to activate the first enzyme, however, the second enzyme may experience only +0.1V because of its different position on the molecular wire. The second enzyme is thus not activated. To activate only the second enzyme, a +0.4V pulse could be applied at first. This results in +0.3V at the first enzyme, and in +0.2V at the second enzyme, which activates both enzymes. A second pulse of -0.3V may then result in the deactivation of the first enzyme, which experiences -0.2V, but does not lead to deactivating the second enzyme, which experiences only -0.1V.

In an implementation of the device, the device comprises: one or more synthesis pixels, wherein each synthesis pixel comprises a synthesis well formed on the support substrate and a respective electrically controlled element (for instance, and electrode element comprising at least one electrode pair) arranged in the synthesis well; electrical multiplexing and/or stimulation circuitry arranged on and/or in the support substrate; a fluidic reservoir comprising a variety of different molecules for interacting with different enzymes, for example, different specific nucleotides; and wherein the multiplexing and/or stimulation circuitry is configured to individually apply the first and/or second voltage profile to each electrically controlled element.

Each synthesis pixel can be used to perform synthesis (e.g., DNA synthesis) using one or more enzymes (e.g., polymerases).

A second aspect of this disclosure provides a method of controlling enzymatic activity using a device according to the first aspect or any implementation thereof, wherein the method comprises: applying a first and/or second voltage profile to an electrically controlled element of the device to change an enzymatic activity of respectively the first enzyme and/or the second enzyme.

This change in enzymatic activity is due to a change in the redox state of the redox-active component of the first enzyme and/or the second enzyme in response to respectively applying the first voltage profile and/or the second voltage profile.

In an implementation of the method for DNA synthesis, wherein the method comprises: activating the polymerase by applying the first voltage difference between the first and second electrode of the device; monitoring the current flowing over the molecular structure comprising the polymerase, and detecting a successful incorporation by the polymerase of a nucleotide into a primer strand based on an indicative current pattern; and deactivating the polymerase, when detecting at least one successful incorporation, by applying the second voltage difference between the first and second electrode.

The method of the second aspect achieves the same advantages described above for the device of the first aspect.

Advantageously, the cyclic nature for writing DNA may performed *in situ* at each of one or more synthesis pixels of the device, by controlling and monitoring the respective polymerase activity-rather than by synchronizing the reactions at selective sites by means of cyclic reagent flushing.

This disclosure in summary proposes a transition from controlling nucleotide incorporation via sequential fluidic delivery to controlling the nucleotide incorporation directly at each synthesis site using electrochemistry. This enables a "one-pot" approach where all necessary reagents are continuously present in a fluidic reservoir to which the device is exposed. It may be achieved by tightly controlled spatiotemporal specificity of enzymatic reactions.

The disclosure further proposes using engineered polymerases, whose activity (reaction rate) and potentially specificity can be controlled electrochemically in real-time and ideally monitored. These enzymes are attached via the conductive molecular wires to the electrodes, which may be integrated into a CMOS chip. The electrochemical potential applied to the electrodes is able to modulate the enzyme's state (e.g., via a redox-active component). An example of the enzyme is a DNA primase (p58c), whose DNA binding affinity is redox-dependent.

### BRIEF DESCRIPTION OF THE DRAWINGS

The above-described aspects and implementations are explained in the following description of embodiments with respect to the enclosed drawings:
- FIG. 1A: shows exemplary variants of a device according to this disclosure.
- FIG. 1B: shows further exemplary variants of a device according to this disclosure.
- FIG. 2: illustrates a concept of this disclosure, according to which a device has direct electrical control over an enzyme (e.g., a polymerase) which is tethered via a conductive molecular wire between two electrodes.
- FIG. 3: shows an exemplary layout of a synthesis pixel of a device according to this disclosure.
- FIG. 4: shows a method for controlling enzymatic activity according to this disclosure.
- FIG. 5: shows a simplified diagram of a *de novo* DNA synthesis procedure implemented by a method according to this disclosure.

### DETAILED DESCRIPTION OF EMBODIMENTS

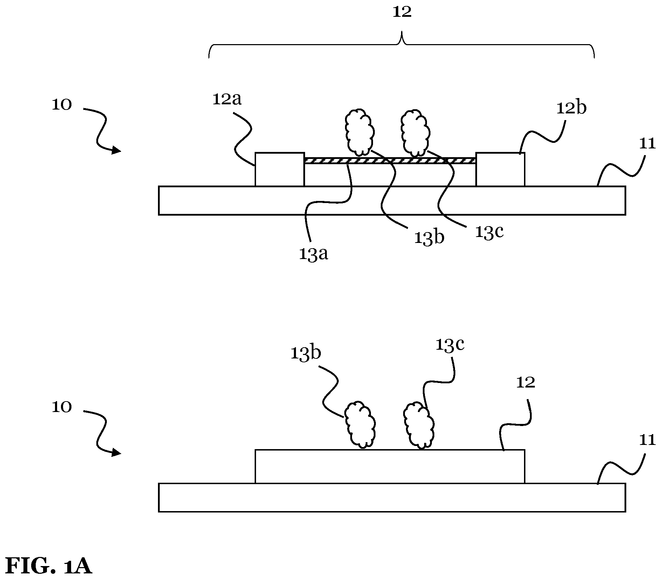

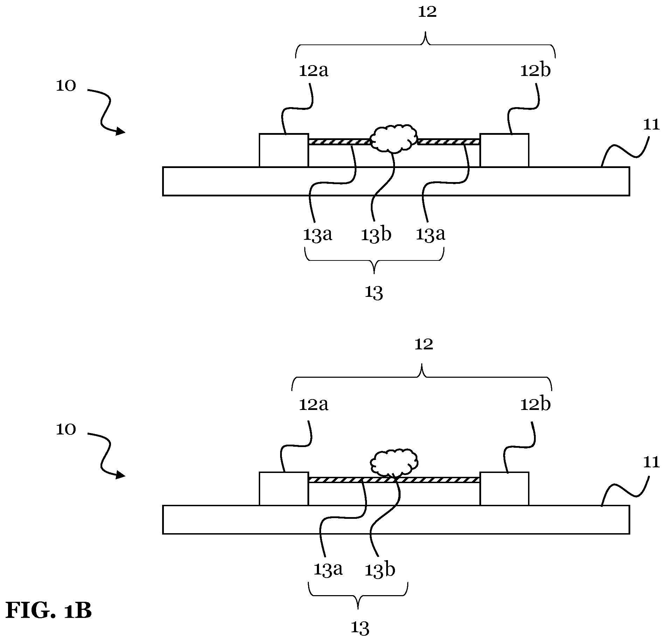

FIGs. 1A and 1B show a device 10 according to this disclosure, in particular, show different exemplary variants of this device 10. The device 10 comprises in all variants at least two enzymes, and is configured to control the enzymatic activity of these enzymes. The device 10 may comprise electrodes, circuits, and other components for electrically controlling the enzymatic activity of the enzymes, wherein all components can be integrated into a chip, for instance, into a CMOS chip. For a CMOS integration, the device 10 may comprise CMOS control hardware, like multiplexers, amplifiers, voltage sources, or the like. The enzymatic activity of the enzymes can be controlled individually.

In particular, the device 10 comprises in all variants a solid support substrate 11, which may be a CMOS substrate (e.g., may be silicon-based). The device 10 further comprises an electrically controlled element 12, which is arranged on the support substrate 11. The electrically controlled element 12 may be an electrode, electrode pair, or similar element that allows applying a voltage or voltage profile to a molecule or enzyme. In FIG. 1A, in the first shown variant (top picture) the electrically controlled element 12 comprises an electrode pair of a first electrode 12a and a second electrode 12b. These two electrodes 12a, 12b of the electrode pair are separated by a gap, for example, a gap of nanometer scale. This is the same in both exemplary variants of FIG. 1B. In the second variant shown in FIG. 1A (bottom picture), the electrically controlled element 12 comprises a single electrode.

The device 10 further comprises in all variants at least a first enzyme 13b and a second enzyme 13c, which are electrically coupled to the electrically controlled element 12. Each enzyme 13b, 13c comprises at least one redox-active component.

In the second variant of FIG. 1A, the first enzyme 13b and the second enzyme 13c are (e.g., directly) attached to the electrode.

In the other variants of FIG. 1A and 1B, the device 10 comprises a molecular structure 13 that bridges the gap between the electrodes 12a and 12b. The molecular structure 13 is thereby attached to the first electrode 12a on one side of the gap, and to the second electrode 12b on the other side of the gap. The molecular structure 13 comprises at least one conductive molecular wire 13a, and the first enzyme 13b and/or the second enzyme 13c attached to at least one molecular wire 13a.

For example, in the variant shown in FIG. 1A (top picture), the first enzyme 13b and the second enzyme 13c are attached to the same molecular wire 13a. More than two enzymes may be attached to the molecular wire 13a.

For example, in one variant (bottom picture) shown in FIG. 1B, the molecular structure 13 comprises one molecular wire 13a that bridges the gap between the first and second electrode 12a, 12b, and the first enzyme 13b (or the second enzyme 13c) is attached to the molecular wire 13a between the first electrode 12a and second electrode 12b. In this case, the device 10 comprises another electrode pair with another molecular wire bridge, to which the second enzyme 13c is likewise attached (not shown).

In another variant (top picture) shown in FIG. 1B, the molecular structure 13 comprises two molecular wires 13a, wherein each molecular wire 13a is connected with one of its ends to one of the first electrode 12a and the second electrode 12b, and wherein the first enzyme 13b (or could also be the second enzyme 13c) is attached between the first electrode 12a and the second electrode 12b to the respective other ends of the molecular wires 13a. That is, the two molecular wires 13a (may be considered as two segments of the molecular wire 13a) sandwich the enzyme 13b. In this case, the device 10 comprises another electrode pair further molecular wire bridge parts, to which the second enzyme 13c (or the first enzyme 13b) is likewise attached (not shown).

In all variants, each enzyme 13b, 13c comprises a respective redox-active component. A first voltage profile applied to the first enzyme 13b causes a change in the enzymatic activity of the first enzyme 13b, which is due to a change in the redox state of the redox-active component of the first enzyme 13b. The change in the redox state of the redox-active component of the first enzyme 13b is caused by applying the first voltage profile. A second voltage profile applied to the second enzyme 13c, which is different to the first voltage profile, causes a change in the enzymatic activity of the second enzyme 13c, which is due to a change in the redox state of the redox-active component of the second enzyme 13c. The change in the redox state of the redox-active component of the second enzyme 13c is caused by applying the second voltage profile.

For example, if a first voltage difference is applied, as a part of the first voltage profile, between the first and the second electrode 12a, 12b in the variants with electrode pair, the redox-active component of the first enzyme 13b may be oxidized. If a second voltage difference is applied, as part of the first voltage profile, between the first and second electrode 12a, 12b, the redox-active component of the first enzyme 13b may be reduced (this may be vice versa). This respectively causes a change in the enzymatic activity of the first enzyme 13b. The same can be done with the second enzyme 13c. The first and second voltage profile may be applied to the first and second enzyme 13b, 13c, respectively, even if they are both attached to the same electrode or same molecular wire.

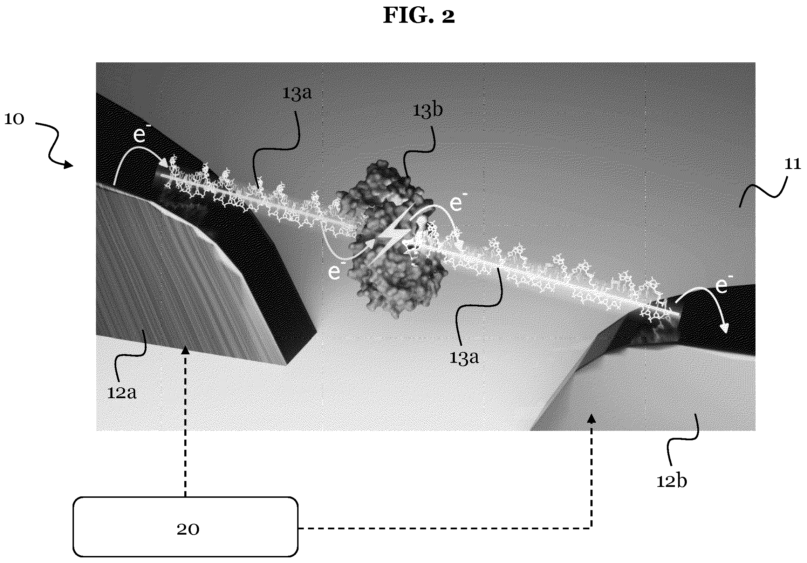

FIG. 2 illustrates the concept of electrically controlling the enzymatic activity of an enzyme (in the following, the first enzyme 13b is used for the purpose of explaining, but it works with the second enzyme 13c as well) in an exemplary variant of the device 10. The first enzyme 13b may be a polymerase (e.g., protein, center), and may be tethered via the conductive molecular wire(s) 13a (e.g., being DNA or peptide-based) between the two electrodes 12a, 12b in the variant with electrode pair. The electron (e⁻) flow (current) may be modulated or controlled by the electrode potential, which influences the enzyme's 13b activity for applications like DNA synthesis or other examples. The molecular wire 13a serves both as a tether and as a conduit for the electron transfer or modulation.

This direct electrical control and interrogation of single enzymes, for example, involved in synthesis, can provide a more flexible, scalable, and programmable approach than using fluorescence or ionic current sensing techniques to transduce biological into electrical signals, which are relatively difficult to scale and parallelize for complex, programmable synthesis. This disclosure particularly focuses on applying the illustrated concept to *de novo* DNA synthesis, but the concept can also be used for controlling other enzyme-based reactions.

In the following, details of implementation for the device(s) 10 shown in FIG. 1 and FIG. 2 are described.

The first enzyme 13b may be an engineered enzyme, for instance, a redox-engineered enzyme. In particular, the first enzyme 13b may be a polymerase, which may be engineered. Standard DNA polymerases rely on template base-pairing for fidelity. For a "one-pot" de novo synthesis proposed by this disclosure (i.e., adding arbitrary sequences without a template strand in the solution without temporal solution exchange) the polymerase may be designed according to certain criteria laid out below.

Firstly, the polymerase may be engineered for a template-independent operation. That is, the polymerase is designed to add nucleotides without relying on a complementary strand present in the fluidic reservoir. Secondly, it may have nucleotide specificity. That is, it may exhibit a near-exclusive specificity for incorporating only one type of nucleotide (i.e., A, C, G, or T). Thirdly, it may have a controllable activity. For instance, its polymerization activity may be reversibly switched on and off with high efficiency and specificity using an external signal (e.g., an electrochemical potential). These criteria can be achieved through enzyme engineering, potentially via several routes.

For example, for achieving the template-independence and specificity, a short synthetic single-stranded "pseudo-template" may be permanently attached within the polymerase's active site. This pseudo-template may consist of universal bases such as inositol (I) followed by a single natural base (e.g., 3'-IIIIA-5') designed to preferentially bind and direct the incorporation of its Watson-Crick complement (T in this example).

As another example, for achieving the controllable activity, a redox-sensitive DNA aptamer may be attached to the polymerase. The aptamer may contain a redox-active group (e.g., methylene blue). The aptamer may be designed to bind and block the polymerase's active site in one redox state (e.g., oxidized, +0.5 V) and release upon changing the redox state (e.g., reduced, -0.5 V), thereby activating the enzyme 13b. This mechanism is analogous to a thermal activation using aptamers, but uses electrochemical potential as trigger. A high reversibility (e.g., target >100 cycles) and blocking efficiency (e.g., target >99.99%) may be benefits for this approach.

In an alternative approach, an engineered native redox site may be obtained. For example, using rational design methods, polymerases may be engineered that naturally possess or are modified to include redox-active sites (like the [4Fe-4S] cluster in DNA primase), which is directly linked to their catalytic activity or processivity. This may avoid aptamer complexity.

It is also possible to leverage engineered variants of terminal deoxynucleotidyl transferase (TdT), which are known for template-independent activity, for example, by modifying them for controllable nucleotide-specific incorporation, possibly using cleavable conjugates or redox control elements. The aptamer-based control combined with an internal template has benefits, however, due to the modularity it offers.

In the following, an exemplary configuration for a synthesis pixel of the device 10 is described, for example, for performing a DNA synthesis. The device 10 may comprise a whole synthesis chip featuring a dense array of reaction sites (i.e., multiple "synthesis pixels"). Target densities may range from >1K to potentially >1M sites/cm², aiming for compatibility with applications like DNA data storage. Each synthesis pixel may be configured to synthesize at least one DNA strand arbitrary sequence and length (e.g., respective targets may be: >100 to >10000 nucleotides per strand).

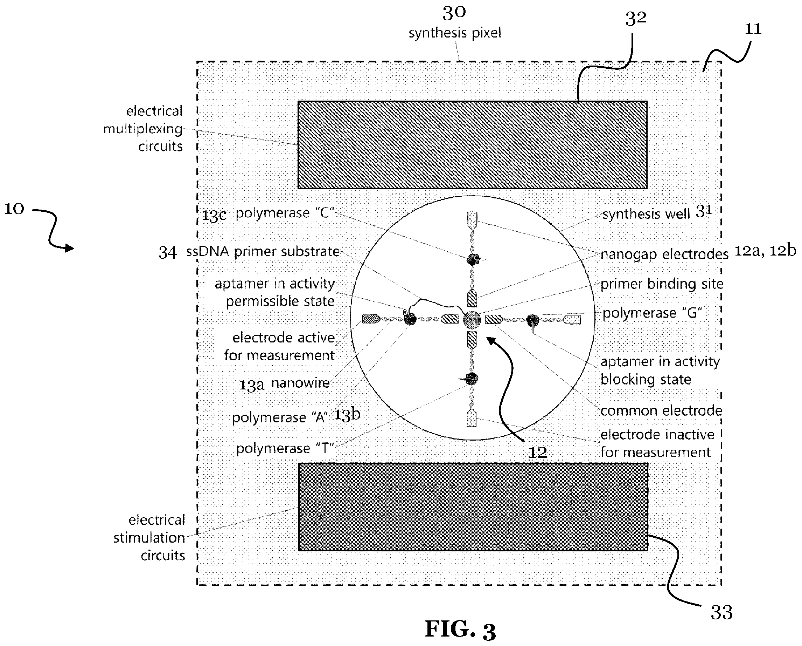

FIG. 3 shows an exemplary layout of a synthesis pixel 30. The synthesis pixel 30 may comprise a synthesis well 31 formed on the support substrate 11, and a respective electrodes of the electrically controlled element 12 comprising at least one electrode pair. Usually, multiple electrode pairs are arranged in the synthesis well 31 of the synthesis pixel 30.

Further, each synthesis pixel 30 comprises electrical multiplexing circuitry 32 and/or stimulation circuitry 33, which is arranged on and/or in the support substrate 11. It can be partly arranged on the surface of the substrate 11, and/or partly arranged embedded in the substrate 11. The multiplexing and/or stimulation circuitry 32, 33 may be configured to-based on a control of the controller 20-individually apply the first and/or second voltage difference between each electrode pair and/or to individually measure the current flowing over each molecular structure 13.

The synthesis pixel 30 further comprises a fluidic reservoir comprising a variety of different molecules for interacting with different enzymes, including the first enzyme 13b and the second enzyme 13c or even more enzymes, for example, different specific nucleotides may be present in the fluidic reservoir. The fluidic reservoir may be shared by multiple synthesis pixels 30 of the device 10.

The synthesis well 31 of the synthesis pixel shown as example in FIG. 3 includes four electrode pairs. Each electrode pair is connected to one molecular structure 13 comprising at least one conductive molecular wire 13a and a polymerase as enzyme (e.g., the first enzyme 13b may be the polymerase "A" and the second enzyme 13c may be the polymerase "C"). The polymerases have different affinities for a specific nucleotides. In particular, the illustrated synthesis well 31 contains four immobilized, individually addressable polymerase units (A, C, G, T), each being tethered by a molecular wire 13a between a specific gap of an electrode pair, wherein the electrode pairs may comprise an electrode 12a for active measurement and a (potentially common) electrode 12b. Each polymerase 13b has a redox-controlled aptamer for activity switching (shown in blocking/permissible states in FIG. 3). A single-stranded DNA primer substrate (short "primer strand") is additionally immobilized centrally on the substrate 11. The dimensions of the pixel 30 may be designed to enhance density while preventing crosstalk.

The synthesis pixel 30 may contain multiple, even more than four electrodes-e.g., at least N+1 for N distinct controllable enzymes (likely N=4 for A, C, G, T polymerases), and potentially plus others for deblocking or error correction (i.e., five or more electrode pairs per pixel 30). An arbitrary voltage pattern can be applied to these electrode pairs and may exert spatiotemporal control over enzymatic activity. A common configuration may involve N specific electrodes 12a and one common counter/reference electrode 12b within or near the pixel 30.

The enzymes 13b, 13c-in this example polymerases, but could be other enzymes as well-are tethered via a molecular wire 13a that physically and electrically connects the two or more electrodes pairs (arranged horizontally or vertically). The molecular wire 13a can be DNA-based (potentially metallized or doped for better conductivity) or protein-based (e.g., conductive peptides). DNA offers programmability via self-assembly but may have lower intrinsic conductivity than engineered protein wires. Protein wires may offer better charge transport but less straightforward functionalization.

Different tethering configurations are possible, as already shown in FIG. 1A and 1B. The enzymes 13b, 13c can be attached "on top" of the molecular wire 13a, or can form a "sandwich", wherein the respective enzyme 13b, 13c bridges the molecular wire segments connected to the electrodes 12a, 12b, respectively. The "sandwich" configuration is beneficial for monitoring current modulations through the enzyme during its catalytic cycle.

The electrode materials of the electrodes 12a, 12b may be configured to facilitate efficient electron transfer with the organic/biological components (molecular wire 13a, redox groups, etc.), and may be configured to be compatible with CMOS back-end-of-line (BEOL) processing (e.g., Pt, TiN, functionalized graphene or MX₂), and may further allow modification with self-assembled monolayers (SAMs) for stable, specific biomolecule attachment. To minimize noise, crosstalk, and unwanted side reactions (e.g., electrolysis of water, non-specific adsorption), the exposed conductive surface area may be minimized, ideally only at the point of connection to the molecular wire 13a within the gap.

The path of the electrons (i.e., the electric current) may be as follows within a synthesis pixel 30 configured according to the "sandwich" configuration:
▪ CMOS circuit (driving and/or sensing circuitry)
▪ First electrode 12a
▪ Inorganic-organic interface chemistry (e.g., thiol-gold bond)
▪ Molecular wire 13a (first segment)
▪ Organic-enzyme interface chemistry
▪ Intra-enzyme 13b electron pathway (potentially involving conformational changes modulating conductivity)
▪ Enzyme-organic interface chemistry
▪ Molecular wire 13a (second segment)
▪ Organic-inorganic interface chemistry
▪ Second electrode 12b (potentially a common electrode)
▪ CMOS circuit (sensing and/or driving circuitry)

Operation modes and activity monitoring of the device 10 can be implemented as follows.

To control enzymatic activity with a single or multi-electrode pairs, specific potentials (e.g., ±0.5 V) may oxidize or reduce the respective redox-active components (e.g., in the aptamer) of the enzymes 13b, 13c, switching the enzymes' activity on or off, respectively. An auxiliary (counter) electrode may ensure charge balance.

To monitor (e.g., in a multi-electrode "sandwich" approach), a smaller, non-perturbing potential difference may be applied between the electrodes 12a, 12b of the electron pair(s), to allow measurement of the electrical current flowing through the tethered respective molecular structure 13 (enzyme-wire assembly). Conformational changes in the first enzyme 13b and/or second enzyme 13c (e.g., polymerase during nucleotide binding and incorporation) are expected to modulate the conductivity of this molecular junction, resulting in detectable current fluctuations. These fluctuations may serve as a real-time report on the enzymatic activity, for example, synthesis progress.

Regarding sensitivity and feedback, the magnitude and signal-to-noise ratio (SNR) of these single-molecule conductance changes are critical challenges. Success relies on detecting these potentially small signals reliably. The real-time monitoring thus may enable a feedback loop. The device 10 can detect successful incorporation(s) and then immediately apply the "off" signal (e.g., oxidative potential) to the polymerase 13b, ensuring single-base resolution or addition of a precise number of bases.

As an alternative to achieving reliable single-step incorporation solely through polymerase on/off control, the device 10 can also be augmented by using reversible terminator nucleotides (e.g., with a 3'-O-blocking group). In this scenario, the polymerase may incorporate one blocked nucleotide and stop. A separate, dedicated "deblocking" enzyme (also potentially electrochemically controlled via its own electrode/wire system within the pixel 30) would then be activated to remove the blocking group, preparing the strand for the next cycle. This provides a robust mechanism for single-step control.

For CMOS integration of the device 10, CMOS control hardware (e.g., multiplexers, amplifiers, voltage sources) can be integrated directly underneath the synthesis pixel 30 (maximizing speed and signal integrity but potentially limiting density) or moved outside the main "synthesis zone" (maximizing density but potentially introducing routing challenges and noise). The optimal layout depends on trade-offs between density, speed, noise, and fabrication complexity.

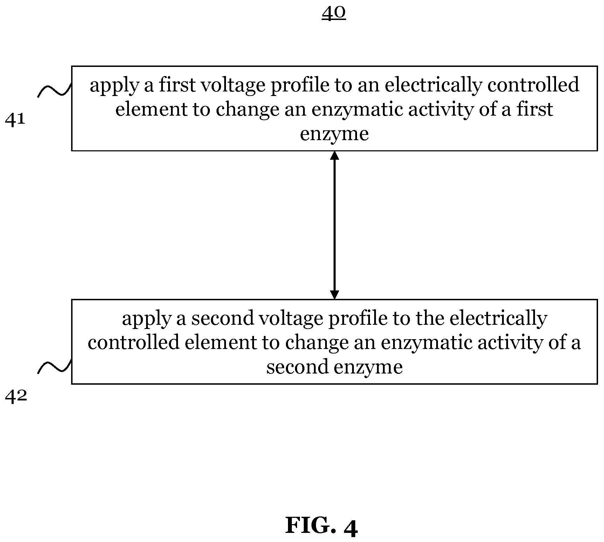

FIG. 4 shows a flow chart of a method 40 of controlling enzymatic activity using the device 10. Generally, the method 40 comprises a step 41 of applying the first voltage profile to the electrically controlled element 12 of the device 10. This causes a change in the enzymatic activity of the first enzyme 13b, which is due to a change in the redox state of the redox-active component of the first enzyme 13b. The method 40 further comprises a step 42 of applying the second voltage profile to the electrically controlled element 12 of the device 10. This causes a change in the enzymatic activity of the second enzyme 13b, which is due to a change in the redox state of the redox-active component of the second enzyme 13c.

The method may also comprise applying a third voltage profile to the electrically controlled element 12 of the device 10, to determine the enzymatic activity of the first enzyme 13b and/or the second enzyme 13c. For example, by measuring a current flowing over a molecular structure 13 comprising the first and/or second enzyme 13b, 13c. Alternatively, the activity of the first and/or second enzyme 13b, 13c may be optically determined and monitored.

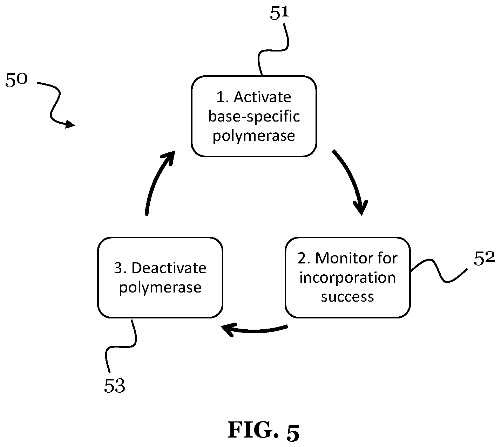

With respect to FIG. 5, a plausible mode of operation for a single synthesis pixel 30 synthesizing an arbitrary DNA sequence, starting from a pre-immobilized primer strand, is described in the following.

Step 51, activate polymerase "A" (first enzyme 13b): Apply a reductive potential (e.g., -0.5 V between the electrodes 12a, 12b) specifically to an "A" polymerase's electrode pair. This reduces the redox aptamer, causing it to release from the active site and activate the polymerase. This activation step itself might be detectable electrically.

Step 52, monitor incorporation success: Apply a non-perturbing monitoring voltage (e.g., between -0.2 V and +0.2 V, or a small AC voltage) across the "A" polymerase's gap. Observe real-time current or impedance fluctuations indicative of primer binding and nucleotide incorporation. The feedback loop may count incorporation events or detect cessation of activity. If multiple "A" are required, maintain the activating potential until the target number is reached. The monitoring voltage must be chosen carefully to avoid unintended redox reactions while allowing sensitive current measurement.

Step 53, deactivate polymerase "A": Apply an oxidative potential (e.g., +0.5 V between the electrodes 12a, 12b) to the "A" polymerase's electrode pair. This oxidizes the aptamer, causing it to re-bind and sterically block the active site. This deactivation may also be electrically monitored.

The steps 51-53 may be repeated, for example, for the next required nucleotide (e.g., "C").

All necessary components (e.g., four types of dNTPs, cofactors, ions, buffers, etc.) are present in the "one-pot" solution, i.e. the fluidic reservoir, flowing over the device 10 at sufficient concentrations for diffusion not to be rate-limiting, and are replenished from the bulk. Crosstalk between synthesis pixels 30 and between polymerase stations (electrode pairs) within a pixel may minimized through design (e.g., electrode geometry, insulation, localized electrochemical fields).

Notably, the approach of this disclosure potentially results in a lower synthesis site density compared to some passive chemical methods (e.g., photolithography or "classic" electrochemical DNA synthesis), which may be due to the CMOS control circuitry used per pixel (functional "real-estate"). Current estimates suggest that electrochemical methods may achieve densities around 10⁹ sites/cm², while photolithographic methods aim for 10⁸ sites/cm² or higher. However, the impact on overall cost-effectiveness is definitely mitigated. Reagent cost scales with the flow-cell volume above the device 10, which is determined by the synthesis zone area, not solely the number of sites. Furthermore, complex CMOS logic may potentially be moved away from the synthesis pixel 30 (e.g., underneath or off-chip via 3D integration or chiplet-interposer approaches) to maximize density in the synthesis layer itself, though this adds fabrication complexity.

In summary, this disclosure revolutionizes control over enzymatic activity, in particular, for *de novo* DNA synthesis, by shifting control from bulk fluidics to localized electrochemistry, enabling a "one-pot" process that significantly reduces reagent consumption, enhances synthesis speed, and provides precise, real-time site-specific control and monitoring, paving the way for more efficient, cost-effective, and versatile DNA synthesis technologies.

In the claims as well as in the description of this disclosure, the word "comprising" does not exclude other elements or steps and the indefinite article "a" or "an" does not exclude a plurality. A single element may fulfill the functions of several entities or items recited in the claims. The mere fact that certain measures are recited in the mutual different dependent claims does not indicate that a combination of these measures cannot be used in an advantageous implementation.

## Claims

1. A device (10) for controlling enzymatic activity, the device (10) comprising:
a solid support substrate (11);
an electrically controlled element (12) arranged on the support substrate (11);
wherein at least a first enzyme (13b) and a second enzyme (13c) are electrically coupled to the electrically controlled element (12);
wherein each enzyme (13b, 13c) comprises at least one redox-active component;
wherein a first voltage profile applied to the first enzyme (13b) causes a change in the enzymatic activity of the first enzyme (13b); and
wherein a second voltage profile applied to the second enzyme (13c), which is different to the first voltage profile, causes a change in the enzymatic activity of the second enzyme (13c).

2. The device (10) according to claim 1, wherein
the electrically controlled element (12) comprises an electrode pair of a first electrode (12a) and a second electrode (12b), which are separated by a gap;
a molecular structure (13) bridges the gap and is attached to the first and the second electrode (12a, 12b); and
the molecular structure (13) comprises at least one molecular wire (13a) and the first enzyme (13b) and/or the second enzyme (13c) attached to the at least one molecular wire (13a).

3. The device (10) according to claim 2, wherein
the first enzyme (13b) and the second enzyme (13c) are attached to the same molecular wire (13a); and
the first and the second voltage profile are respectively selected based on the respective position of the first enzyme (13b) and the second enzyme (13c) on said molecular wire (13a).

4. The device (10) according to claim 2 or 3, wherein
the first and/or second voltage profile applied to the first and second electrode (12a, 12b) respectively oxidizes or reduces the redox-active component of the first and/or second enzyme (13b, 13c), which causes a change in the enzymatic activity of the respective enzyme(s) (13b, 13c).

5. The device (10) according to one of the claims 1 to 4, wherein the device (10) further comprises a controller (20) configured to:
apply the first voltage profile and/or the second voltage profile to the first and/or the second enzyme (13b, 13c) via the electrically controlled element (12).

6. The device (10) according to claim 5 and claim 3 or 4, wherein the controller (20) is configured to
apply, as a part of the first voltage profile, a first voltage difference between the first and second electrode (12a, 12b) to pass a current over the molecular structure (13), which oxidizes the redox-active component of the first enzyme (13b);
apply, as a part of the first voltage profile, a second voltage difference between the first and second electrode (12a, 12b) to pass a current over the molecular structure (13), which reduces the redox-active component of the first enzyme (13b); and
apply a third voltage difference between the first and second electrode (12a, 12b) to measure a current flowing over the molecular structure (13), or optically monitor the enzymatic activity of the first enzyme (13b); and
wherein optionally the controller (20) is further configured to
determine a current pattern in the measured current flowing over the molecular structure (13), wherein the current pattern is indicative of the enzymatic activity of the first enzyme (13b).

7. The device (10) according to one of the claims 2 to 6, wherein
the molecular structure (13) comprises one molecular wire (13a) bridging the gap and attached to both the first and second electrode (12a, 12b); and
the first enzyme (13b) and/or the second enzyme (13c) is attached to the molecular wire (13a) between the first and second electrode (12a, 12b).

8. The device (10) according to one of the claims 2 to 6, wherein
the molecular structure (13) comprises two or more molecular wires (13a), each molecular wire (13a) being connected with one of its ends to one of the first and second electrode (12a, 12b); and
the first enzyme (13b) and/or the second enzyme (13c) is attached between the first and second electrode (12a, 12b) to the respective other end of at least one molecular wire (13a).

9. The device (10) according to one of the claims 1 to 8, wherein
the first enzyme (13b) and/or the second enzyme (13c) is a polymerase, for example, a redox-active polymerase comprising a redox-active aptamer.

10. The device (10) according to claim 9, wherein
the polymerase (13b, 13c) has an affinity for a specific nucleotide.

11. The device (10) according to claim 10, wherein
a synthetic single-stranded pseudo-template strand (34) is attached to the polymerase (13b, 13c) within an active site of the polymerase (13a); and
the single-stranded pseudo-template strand (34) comprises a single natural base, which provides the polymerase (13b, 13c) with the affinity for the specific nucleotide.

12. The device (10) according to one of the claims 1 to 11, wherein:
the electrically controlled element (12) is connected to a plurality of enzymes (13b, 13c) comprising the first enzyme (13b) and the second enzyme (13b); and
each enzyme (13b, 13c) comprises a different polymerase with a different affinity for a specific nucleotide than the other polymerases.

13. The device (10) according to one of the claims 1 to 12, comprising:
one or more synthesis pixels (30), wherein each synthesis pixel (30) comprises a synthesis well (31) formed on the support substrate (11) and a respective electrically controlled element (12);
electrical multiplexing circuitry (32) and/or stimulation circuitry (33) arranged on and/or in the support substrate;
a fluidic reservoir comprising a variety of different molecules for interacting with different enzymes (13b, 13c), for example, different specific nucleotides; and
wherein the multiplexing and/or stimulation circuitry (32, 33) is configured to individually apply the first and/or second voltage profile to each electrically controlled element.

14. A method (40) of controlling enzymatic activity using a device (10) according to one of the claims 1 to 13, wherein the method (40) comprises:
applying (41) a first voltage profile and/or a second voltage profile to an electrically controlled element (12) of the device (10), to change an enzymatic activity of respectively the first enzyme (13b) and/or the second enzyme (13c).

15. The method (40) according to claim 14 for DNA synthesis using a device (10) according to the claims 6 and 9, wherein the method (40) comprises:
activating (51) the polymerase (13b, 13c) by applying the first voltage difference between the first and second electrode (12a, 12b) of the device (10);
monitoring (52) the current flowing over the molecular structure (13) comprising the polymerase (13b, 13c), and detecting a successful incorporation by the polymerase (23b) of a nucleotide into a primer strand based on an indicative current pattern; and
deactivating (53) the polymerase (13b, 13c), when detecting at least one successful incorporation, by applying the second voltage difference between the first and second electrode (12a, 12b).
